# EUROPEAN PATENT SPECIFICATION

(11) **EP 2 232 268 B1**
(45) Date of publication and mention of the grant of the patent: **02.05.2012**
(21) Application number: 08860427.7
(22) Date of filing: 11.12.2008
(51) Int. Cl.: G01N 33/68

(54) **USE OF KIR GENES FOR PREDICTING RESPONSE TO THERAPY**
VERWENDUNG VON KIR-GENEN ZUR VORHERSAGE DER REAKTION AUF EINE THERAPIE
UTILISATION DE GÈNES KIR POUR PRONOSTIQUER LA RÉPONSE À UNE THÉRAPIE

(30) Priority: 11.12.2007 GB 0724131
(43) Date of publication of application: 29.09.2010
(73) Proprietor: UNIVERSITY OF ULSTER, County Londonderry BT52 1SA (GB)
(72) Inventor: BERRAR, Daniel, Tokyo 162-0851 (JP); BJOURSON, Anthony, Strabane County Tyrone BT82 9DR (GB); MCGEOUGH, Cathy, Armagh County Armagh BT60 2EB (GB)
(74) Representative: O'Connell, Maura
(86) International application number: PCT/EP2008/010547
(87) International publication number: WO 2009/074326

(56) References cited:
- CHAN M, LO G, RODDY JE, WITT C, KEEN HI, CHRISTIANSEN FT, TAYLOR AL: "Kir genotypes as predictors of response to therapy with DMARDS and anti-TNF agents in rheumatoid arthritis" INTERNAL MEDICINE JOURNAL, vol. 33, no. suppl., 2003, page A74, XP002528298

## Description

### Background

The Killer immunoglobulin-like receptors (KIRs) are the main products of the leukocyte receptor complex gene cluster, located on human chromosome 19q13.4. This family of polymorphic receptors may be either activating (2DS/3DS) or inhibiting (2DL/3DL) in function and modulate natural killer (NK) cell and T-cell function through recognition of human histocompatibility leukocyte antigen (HLA) class I molecules on target cells. The integration of activatory and inhibitory signals transduced by KIR on NK and T-cell subsets plays a role in maintaining a balance between effective immune surveillance and autoimmune reaction. To date, 15 *KIR* genes have been identified, of which the *2DS*/*2DL* types are perhaps the best characterized. Inhibitory KIR2DL2 and KIR2DL3 recognise HLA-C group 1 ligands. However, their corresponding activatory receptor KIR2DS2 demonstrates little or no binding affinity for these ligands. Inhibitory KIR2DL1 recognizes HLA-C group 2 ligands and, similarly, its corresponding activatory receptor KIR2DS1 binds with much less affinity. Individuals may be HLA-C1 or -C2 homozygous (C1/C1, C2/C2) or heterozygous (C1/C2). If an individual lacks the ligand for a particular KIR, then that KIR is usually considered non-functional.

Within the NK receptor repertoire, KIRs are a key receptor family, which regulate NK cell activity. In addition to their innate function, NK cells have the potential to interact with components of the adaptive immune system, including T-cells and dendritic cells. This dual function of NK cells suggests their likely involvement in autoreactive immune responses such as rheumatoid arthritis (RA). KIRs are also present on CD8⁺ T-cells and CD4⁺CD28^{null} T-cells confirming their additional involvement in adaptive immunity.

The precise role for KIR on T-cells however remains unclear. It is speculated that they may support the ability to escape attack from cytotoxic T-cells, which react with autoantigens. Interestingly, the expression of KIR2DL1/2DS1 on a population of CD8⁺ T-cells was found to be remarkably lower in RA patients compared to healthy controls. The down-regulation of inhibitory KIR2DL1 on these T-cells could lower the threshold for initiation of an immune response.

Autoimmune diseases affect millions of people worldwide and, with more than 70 distinct types of pathologies associated with autoimmune processes, represent a wide and fast-moving area of research and clinical interest. Tumour necrosis factor alpha (TNFα), in particular, emerged from these studies as a key inflammatory mediator in the chronic inflammatory process, acting as a pivotal regulator of expression of other pro-inflammatory cytokines such as Interleukin-1 (IL-1) and Interleukin-6 (IL-6). Thus TNFα has become a key target for therapeutic intervention in the autoimmune disease setting. Several biotech-derived drugs targeting TNFα have been licensed in recent years, profoundly changing the therapy of several autoimmune diseases based on inflammation.

In this connection, two distinct forms of biologics targeting TNF have been developed, namely monoclonal antibodies (MAbs) and dimeric soluble TNF receptors. Both types of biologics are approved for clinical use for different indications:

Infliximab (Remicade^{®}) is a chimeric IgG1 Mab that binds to TNFα and TNFβ and lyses TNF-producing cells to neutralise their activity. The U.S. Food and Drug Administration (FDA) have approved it for the treatment of psoriasis, paediatric Crohn's disease, ankylosing spondylitis, Crohn's disease, psoriatic arthritis, rheumatoid arthritis, and ulcerative colitis.

Etanercept (Enbrel^{®}) is a recombinant dimer of human p75 TNF receptor proteins fused and bound to human IgG1 that acts competitively to inhibit the binding of TNF to its cell surface receptor, originally approved in 1998 by the FDA to treat the painful joint swelling and deterioration caused by rheumatoid arthritis. To date, Etanercept has not been shown to be effective in the treatment of Crohn's disease.

Adalimumab (Humira)^{™} is a humanised anti-TNFα MAb that binds with high affinity to TNFα. As of February 2007, adalimumab has been approved for the treatment of rheumatoid arthritis, psoriatic arthritis, ankylosing spondylitis and Crohn's disease. Data have been submitted to the FDA for expanding the label to include the treatment of plaque psoriasis. Replacing murine with human elements has resulted in a reduction in the production of antibodies that neutralise adalimumab.

Rituximab, sold under the trade names Rituxan® and MabThera®, a chimeric monoclonal antibody, is a more recently approved TNF-α based therapy approved for rheumatoid arthritis.

New biological agents such as TNFα antagonists have shown remarkable efficacy in the treatment of diseases such as rheumatoid arthritis but 20-40% of patients do not respond to therapy, and there are currently no clinically useful predictive markers of treatment response. This is compounded by the fact that anti-TNFα drugs are very expensive, costing more than £8000 per patient per annum (in 2007 in the United Kingdom), and patients receive treatment for 3-6 months before their clinical response can be determined. The ability to segregate patients into cohorts that will, or will not, respond to treatment is an urgent requirement. Such a personalized medicine approach where treatments are tailored to the genetic compliment of the individual represents the most cost-effective use of health budgets and avoids preventable side effects for those patients that derive no clinical benefit. Therefore, the ability to predict response to anti-TNFα therapy would find universal utility in the clinical management of patients with autoimmune diseases, such as RA.

It is an object of the present invention to prospectively predict the likely clinical response of patients to therapeutic agents used in TNF therapy, based on the genotype of the patient, in particular, by evaluating the presence, absence, or quantitative level of at least one *KIR* gene, and evaluating the presence, absence or quantitative level of a gene encoding a KIR ligand.

### Summary of the Invention

According to a first aspect of the present invention, there is provided the use of at least one *Killer cell immunoglobulin-like receptor* gene selected from 2D52 and 2DL2; and a gene encoding and KIR ligand selected from HLA-C Group 1 and HLA-C Group 2; for the identification of subjects likely to respond to anti-tumour necrosis factor alpha-based therapy.

According to a second aspect of the present invention, there is provided a method of identifying subjects likely to respond to tumour necrosis factor-based therapy, the method comprising the steps of evaluating the presence, absence, or quantitative level of at least one KIR gene selected from 2D52 and 2DL2 for a subject; or evaluative the presence, absence or quantitative level of an expression product encoded by the gene; evaluating the presence, absence, or quantitative level of a gene encoding and KIR ligand selected from HLA-C Group I and HLA-C Group 2; or evaluating the presence, absence, or quantitative level of an expression product encoded by the gene; and stratifying the subject based on the presence, absence, or quantitative level of the at least one KIR gene or based on the presence, absence, or quantitative level of an expression product encoded by the gene; and the presence, absence, or quantitative level of the gene encoding the KIR ligand or based on the presence, absence, or quantitative level of an expression product encoded encoded by the gene.

For the purposes of this specification, the use is determined by a combination of factors, one or more of which being presence or absence of a gene, or quantitative level of a gene, and another of which being the presence or absence of an expression product encoded by that gene, or quantitative level of an expression product encoded by that gene. The use may be determined by evaluating factors including, but not limited to, the presence or absence of a *KIR* gene, the level of a *KIR* gene, the presence or absence of the gene encoding its corresponding ligand, and the level of the gene encoding its corresponding ligand. Optionally or additionally, the use may be determined by evaluating factors including, but not limited to, the presence or absence of an expression product of a *KIR* gene, the level of an expression product of a *KIR* gene, the presence or absence of its corresponding ligand, and the level of its corresponding ligand.

The *KIR* gene may be evaluated by assessing the haplotype of the subject. By the term "haplotype" is meant a region of a chromosome, comprising a series of genes, in any number or combination. In the present case, the haplotype may comprise any of the *KIR* genes, including activatory *KIR* genes, inhibitory *KIR* genes, *KIR* pseudogenes, and framework *KIR* genes. For the purposes of the present specification, a group B haplotype comprises one or more of the genes selected from *KIR2DL5, KIR2DS1, KIR2DS2, KIR2DS3, KIR2DS5, KIR2DL2,* and *KIR3DS1.* A group A haplotype is defined by the absence of any one of the genes selected from *KIR2DL5, KIR2DS1, KIR2DS2, KIR2DS3, KIR2DS5, KIR2DL2,* and *KIR3DS1.* Optionally, a group A haplotype comprises one or more of the genes selected *from KIR2DS4, KIR2DL1, KIR2DL3, KIR3DL1, KIR3DL2, and KIR 3DL3.*

Optionally, the presence of *2DS2* defines a group B haplotype. Further optionally or additionally, the presence of *2DL2* defines a group B haplotype. Optionally, a group B haplotype is indicative that a subject will respond (is a responder).

Optionally or additionally, in a haplotype comprising at least one activatory *KIR* gene and at least one inhibitory *KIR* gene, the presence, absence, or quantitative level of the at least one *KIR* gene can be evaluated by the difference in the number of activatory *KIR* genes and the number of inhibitory *KIR* genes in a suitable biological sample comprising genetic material from a subject. Further optionally, in a haplotype comprising at least one activatory *KIR* gene and at least one inhibitory *KIR* gene, the number of activatory *KIR* genes being greater than, or equal to, the number of inhibitory *KIR* genes, is indicative that a subject will respond (is a responder).

Optionally, the presence of a Group B haplotype is indicative that a subject will respond (is a responder). Further optionally, the presence of an activatory *KIR* gene is indicative that a subject will respond (is a responder). Still further optionally, the number of activatory KIR genes being greater than, or equal to, the number of inhibitory KIR genes, is indicative that a subject will respond (is a responder).

The at least one *KIR* gene is selected from the group comprising those *KIR* genes that are activating in function, and those *KIR* genes that are inhibiting in function. Optionally, the *KIR* gene is selected from the genes that are activating in function. The activating *KIR* genes may be selected from the group comprising, but not limited to, *2DS2, 2DS4, 2DS1, 2DS3, 2DS5, and 3DS1.* Further optionally, the *KIR* gene is *2DS2.* Optionally or additionally, the *KIR* gene is selected from the genes that are inhibiting in function. The inhibiting *KIR* genes may be selected from the group comprising, but not limited to, *2DL1, 2DL4, 2DL3, 2DL5A, 2DL5B, 2DL2, 3DL2, 3DL1,* and *3DL3.* Further optionally or additionally, the *KIR* gene is *2DL2.* Furthermore, the *KIR* genes may also be selected from the group comprising those *KIR* genes that are activating in function, and those *KIR* genes that are inhibiting in function, in any number or combination. Optionally, the *KIR* genes include *2DS2* and/or *2DL2.*

Optionally, the use is determined by the presence or absence of an activatory the *KIR* gene. Further optionally, the use is determined by the presence or absence of *KIR2DS2*. Still further optionally, the use is evaluated by the presence or absence of the gene encoding HLA-C Group 1.

Optionally or additionally the use is determined by the presence or absence of the expression product of an activatory *KIR* gene. Further optionally or additionally, the use is determined by the presence or absence of an expression product of *KIR2DS2.* Still further optionally or additionally, the use is evaluated by the presence or absence of an expression product of the gene encoding HLA-C Group 1.

Optionally or additionally the use, is determined by the presence or absence of an inhibitory *KIR* gene. Further optionally or additionally, the use is determined by the presence or absence of *KIR2DL2.* Still further optionally or additionally, the use is evaluated by the presence or absence of the gene encoding HLA-C Group 2.

Optionally or additionally, the use is determined by the presence or absence of the expression product of an inhibitory *KIR* gene. Further optionally or additionally, the use is determined by the presence or absence of an expression product of *KIR2DL2*. Still further optionally or additionally, the use is evaluated by the presence or absence of an expression product of the gene encoding HLA-C Group 2.

The corresponding KIR ligands may be selected from the group comprising, but not limited to, HLA-C Group 1, and HLA-C Group 2.

It is envisaged that a genomic-based approach such as PCR-SSOP may be used to determine factors such as carriership of a *KIR* gene, and the haplotype of its corresponding ligand. However, it is appreciated that a diverse range of methodologies can be adapted to screen for such genetic variation analyses (see examples discussed in Syvanen, 2001. Accessing genetic variation: genotyping single nucleotide polymorphisms. *Nature Reviews Genetics*), methodologies that may be employed, include: Quantitative-PCR (QPCR), Tissue Microarray (TMA), Restriction Fragment Length Polymorphism analyses (RFLP), or nucleotide sequence analysis. Most contemporary methods may, as the first step, involve PCR amplification of the appropriate genomic region, or involve reverse transcription of the appropriate mRNA. The PCR product thus obtained may then be subjected to sequence analyses by, for example, standard Sanger nucleotide sequencing, pyrosequencing, or mass spectrometry-based methods (see Tost & Gut, 2002. Genotyping single nucleotide polymorphisms by mass spectrometry. *Mass Spectrometry Reviews,* **21**:6, 3880). Other widely used methods that would be easily adapted include allele specific-hybridization, -ligation, -primer extension, -enzymatic cleavage, or denaturing-HPLC. These examples are not intended to be limiting, and a person skilled in the art will be able to determine an appropriate technique.

Optionally, the use may include evaluating factors such as the protein level expressed by a *KIR* gene, and the protein level of its corresponding ligand.

In addition to specific analyses of nucleic acid sequence, an alternative would be to examine the protein component encoded by such nucleic acid sequences. This could enable molecular typing, using methods such as amino acid sequencing of appropriately isolated protein or using antibodies specific to the HLA and/or KIR epitope(s) of interest. It is envisaged that a proteomic-based approach such as Western blotting may be used to determine factors such as the level of a KIR protein, and its corresponding ligand. However, it is appreciated that any other suitable methodology may be employed, including: Enzyme-linked Immunosorbent Assay (ELISA), or amino acid sequence analysis. These examples are not intended to be limiting, and a person skilled in the art will be able to determine an appropriate technique.

Preferably, a combination of at least one pair of a *KIR* gene and its corresponding ligand is evaluated to determine *KIR* gene presence, absence, or quantitative level. Optionally, other factors such as DAS28 score may also be used in combination to stratify the subject.

The term "DAS28" is intended to include "Disease Activity Score", "DAS-CRP", and "DAS-C-Reactive Protein", and relates to a combined clinical index to measure the disease activity in subjects with RA. The DAS28 index may include 28 non-graded joint count for tenderness and swelling, and may be based on the difference between two DAS28 scores evaluated at separate points in time, the difference indicating an improvement or decline in clinical response.

Optionally or additionally, the use is determined using other factors such as DAS28 score.

Preferably, the use is determined by the presence or absence of the *KIR* genes selected from *2DS2* and *2DL2,* and using the DAS28 score.

The interactions of the two domain KIRs are much better defined than those of the remaining KIRs. KIR3DL1 interacts with HLA-B molecules with a Bw4 epitope, whereas KIR3DL2 reacts with particular HLA-A proteins and KIR2DL4 is believed to contact HLA-G molecules.

TNF-based therapy is any therapeutic intervention targeting TNF signaling. Optionally, the therapeutic intervention comprises activating TNF signalling using, for example, TNF agonists. Preferably, the therapeutic intervention comprises inhibiting TNF signaling. More preferably, the therapeutic intervention comprises inhibiting TNFα signalling using, for example, TNFα antagonists

TNFα antagonists (such as etanercept, infliximab and adalimumab) are indicated for inflammatory conditions in which TNFα is a critical mediator of the disease pathogenesis. These include, but are not limited to plaque psoriasis, psoriatic arthritis, juvenile rheumatoid arthritis, ankylosing spondylitis, Crohn's disease and ulcerative colitis.

Optionally, a model can be used to classify a subject. Preferably, the model is based on a predictive analytics method. Preferably, the predictive analytics method is a statistical model. More preferably, it is based on a linear classifier. Most preferably, it is based on a Fisher's Linear Discriminant Analysis (LDA). Although it will be seen, that any predictive analytics technique may be utilised in the process of designing and/or refining the model.

Preferably, the model calculates the posterior probability of a given outcome.
Preferably, the given outcome is that a subject will respond (is a responder).

Preferably, the posterior probability is used to make a decision.

Preferably, the upper critical value is 0.65. Although, it will be seen that the upper critical value can be altered based on the refinement of the model.

For the purposes of the present specification, it is understood that this invention is not limited to the specific methods, treatment regimens, or particular procedures, which as such may vary. Moreover, the terminology used herein is for the purpose of describing particular embodiments and is not intended to be limiting.

By the term "subject", is meant an individual. Preferably, the subject is a mammal. More preferably, the subject is a human.

As used throughout, KIR nomenclature such as "2DS2" is intended to be synonymous with the nomenclature "KIR2DS2".

### Brief Description of the Drawings

An embodiment of the invention will now be described, by way of example with reference to the accompanying drawings, in which:
**Figure 1** illustrates the effect of KIR2DS2 and HLA-C genotypes (I-IV) on clinical response;
**Figure 2A** illustrates differences in DAS28 scores pre- and post-treatment of KIR2DS2 and HLA-C genotypes (I-IV);
**Figure 2B** illustrates differences in DAS28 scores pre- and post-treatment between two KIR2DS2 and HLA-C genotypes (I & IV);
**Figure 3** illustrates a receiver operating characteristic (ROC) of the classifier KIR2DS2;
**Figure 4** is a schematic diagram illustrating a decision support model, which encapsulates the linear discriminant classifier; and
**Figure 5** illutrates the frequency of KIR haplotypes in rheumatoid arthritis (RA) patients and controls.

### Patients and Methods

### Patients

The patient group comprised 70 unrelated Northern Irish Caucasians with chronic rheumatoid arthritis. Each subject was a patient attending the Rheumatology department of Musgrave Park Hospital, Belfast, Northern Ireland, United Kingdom. All patients fulfilled the American College of Rheumatology 1987 revised criteria for RA and had active disease as indicated by a disease activity score in 28 joints (DAS28) of >3.2. There was no significant difference in the distribution of age or sex between responders and non-responders.

Sixty-four patients were assigned to anti-TNF-α treatment (adalimumab, etanercept or infliximab) when at least two other disease modifying antirheumatic drugs (DMARDs) had failed. Forty patients received adalimumab, 15 etanercept and 9 infliximab. In most cases, methotrexate (MTX) was maintained as a combination therapy and patients were allowed to continue oral glucocorticoids. Peripheral blood samples were collected from each patient at time 0. Following three months of treatment, the patients were assigned a responder or non-responder status based on their DAS28 score improvement. See Table 1.

**Table 1. Clinical and demographic characteristics of RA patients.**

| *P*-values determined by student's t-test compare responder and nonresponder to anti-TNF-α therapy groups. Pre-DAS28 scores were calculated at time 0, before beginning anti-TNF-α treatment, post-DAS28 scores are taken three months later. | | | | |
|---|---|---|---|---|
| | **Responders** | **Non-responders** | **All** | **P-value** |
| **Total** | 34 | 30 | 64 | - |
| **Mean age (years)** | 57.97 (range, 35-75) | 54.53 (range, 27-73) | 56.33 (range, 27-75) | 0.21 |
| **Female/Male** | 28/6 | 26/4 | 54/10 | 0.74 |
| **Mean pre-DAS28 score** | 6.21 (range, 4.08-8.96) | 5.43 (range, 3.75-7.38) | 5.85 (range, 3.75-8.96) | 0.004 |
| **Mean post-** | 3.64 (range, | 5.61 (range, | 4.53 (range, | 4.2×10⁻⁸ |
| **DAS28 score** | 1.18-6.15) | 3.49-7.27) | 1.18-7.27) | |

Within the RA population 64 of 70 patients had clinical outcome data available. Within this group of 64 patients (described in Table 1), 34 were responders (53.0 %) and 30 were nonresponders (47.0 %). The mean DAS28 score of responders (6.21) at baseline was significantly higher than those of nonresponders (5.43) at this time point (*P* = 0.004).

A control population of 100 randomly selected, unrelated healthy Northern Irish Caucasians were included for comparison purposes. The healthy control blood samples were collected at Northern Ireland Blood Transfusion Services, Belfast, Northern Ireland, United Kingdom. All participants in the study gave written informed consent and the study had local ethical committee approval.

### KIR Genotyping

Genomic DNA was extracted from peripheral blood lymphocytes using the salting out method as previously described in Miller SA, Dykes DD, Polesky HF. A simple salting out procedure for extracting DNA from human nucleated cells. Nucleic Acids Res 1988;16:1215. DNA was typed for the presence or absence of framework KIR genes: KIR2DL4, KIR3DL2, KIR3DL3, and KIR3DP1, six activatory KIR (KIR2DS1, KIR2DS2, KIR2DS3, KIR2DS4, KIR2DS5, and KIR3DS1) and five inhibitory KIR (KIR2DL1, KIR2DL2, KIR2DL3, KIR3DL1, KIR2DL5). KIR2DP1 was also included in the typing. KIR genotyping was performed using the PCR primers and probes of the PCR sequence specific oligonucleotide probe (PCR-SSOP) method previously described in Middleton D, Williams F, Halfpenny IA. KIR genes. Transpl Immunol 2005;14:135-42. Positive controls of known KIR genotype, collectively incorporating all of the KIR genes, were included in the typing procedure. PCR reactions were used to amplify both the D1/D2 and the transmembrane/cytoplasmic (T/C) regions of the KIR genes. In each reaction, 0.1 µg DNA was amplified in a 100 µl reaction containing 10 µl 10 x reaction buffer (containing 16 mM (NH₄)₂SO₄, 67 mM Tris-HCl pH 8.8 at 25 °C, 0.1 % Tween-20), 2 mM MgCl₂, 0.2 mM dNTP, 0.2 µM of each forward and reverse primer (Table 2), and 3 U *Taq* DNA polymerase (Bioline, Ireland).

**Table 2. Primers for PCR-SSOP genotyping.**

| KIR domain 1 and 2, and transmembrane/cytoplasmic domain primers used in PCR-SSOP are listed. X is the universal base inosine. | | | | |
|---|---|---|---|---|
| **Region** | **Primers** | **5'- Sequence -3'** | **Position** | **Product** |
| D1/D2 | DIF | AGAGAXXGTCATCCTGCA(A/G)TGTTGGTC | Ex 4 56-82 | 2100 bp |
| | D2RR2 | CTCACCTXTGAC(G/A)GAAACAAGCAGTGG | Int 5-273 Ex 5 | |
| | | | | |
| T/C | TMF1 | GCTGTGATTAGGTACTCAGTGGCC | Ex 7 21-44 | 950 bp |
| | TMF2 | GTTCTGATTGGGACCTCAGTGGTC | Ex 7 21-44 | |
| | TMF3 | ATTCTGATTGGGACCTCAGTGGCT | Ex 7 21-44 | |
| | DmcytR | TTGAGACAGGGCT(G/A)TTGTCTCC | Ex 9 232-211 | |
| | | | | |
| HLA-C | 5Cin1-61 | AGCGAGG(G/T)GCCCGCCCGGCGA | Int 1 42-61 | 937 bp |
| | 3BCIn3-12 | GGAGATGGGGAAGGCTCCCCACT | Int 3 35-12 | |

PCR conditions for the D1/D2 amplification consisted of an initial denaturation at 96 °C for 5 minutes, 30 cycles of 96 °C for 1 minute, 60 °C for 30 seconds, 72°C for I minute and a final extension step at 72 °C for 10 minutes (9700 thermal cycler, Applied Biosystems). The T/C amplification was performed with the same conditions increasing the number of cycles to 38 and the cycling extension time to 90 seconds. Amplified products (2.1 Kb) were visualised on a 1.5 % agarose gel at 150 V for 20 minutes. Products were then dot-blotted onto nylon membranes in an 8 x 12 format. Hybridisation of KIR specific digoxigenin-labelled probes was performed. Probe sequences and hybridisation conditions are given in Table 3. Positive probe reactions were determined through chemiluminescent detection and captured on x-ray film. Positive controls of known KIR genotype, collectively incorporating all of the KIR genes were included in the typing procedure.

**Table 3. Sequence-specific oligonucleotide probes used for KIR and HLA-C1/C2 identification.**

| | | | | |
|---|---|---|---|---|
| The 19 KIR probes are those described by Middleton D, Williams F, Halfpenny IA. KIR genes. Transpl Immunol 2005;14:135-42. Probe 2DL1-D1 is in anti-sense direction. HLA-C probes C293 and C291 are taken from Williams F, Meenagh A, Patterson C, Middleton D. Molecular diversity of the HLA-C gene identified in a Caucasian population. Hum Immunol 2002 63:602-13.. Positions are taken from ATG start codon. Probe concentrations are given in picomoles (pm). | | | | |

| **Region** | **Probe** | **5'- Sequence -3'** | **Wash temp (°C) / temp concentration (pm)** | **Position** |
|---|---|---|---|---|
| D1 | Ig2 3DL2 | TACAGATGTTATGGTTCT | 48°C/20pm | Ex 4 222-239 |
| | Ig2 2DS2 | GGGGAAGTATAAGGACA | 48°C/30pm | Ex 4 119-135 |
| | Ig2 2DS4 | TTCTCCATTGGTCCCAT | 48°C/20pm | Ex 4 183-199 |
| | Ig2 2DS3 | CTATCAGTTTTCAGCTCC | 52°C/20pm | Ex 4 254-271 |
| | Ig2 Z (2DP1) | CCATGATGGAAGACCTG | 52°C/30pm | Ex 4 196-212 |
| | Ig2 2DS5a | ACGTTTAACCACACTTTG | 50°C/20pm | Ex 4 123-140 |
| | Ig2 X (3DP1) | GAAGTTTAATGACACTTTG | 50°C/20pm | Ex 4 122-140 |
| | Ig2 3DL3 | CACGATGCGGGTTCCCAG | 60°C/20pm | Ex 4 159-276 |
| | 2DL1-D1^{a} | AGCTGACACCTGATAGG | 58°C/20pm | Ex 4 253-269 |
| | Ig2 2DS1agg | CCATCAGTCGCATGAGGCAAG | 66°C/100pm | Ex 4 187-207 |
| | Ig2 2DS1AgA2 | CCATCAGTCGCATGAAGCAAG 64°C/50pm | | Ex 4 187-207 |
| D2 | Ig1 2DS4del | CTGCAGCT-22bp-CCATCTAT | 48°C/20pm | Ex 4 74-111 |
| | Ig1 2DL2 | CCATGAATGTAGGTTC | 46°C/20pm | Ex 4 128-143 |
| | | | | |
| T/C | NK1 2DL1 | TCTGCAGGAAACAGAAC | 50°C/20pm | Ex 8 24-40 |
| | NK 2DL5 | CATTGCTGCTGCTCCA | 50°C/20pm | Ex 7 81-96 |
| | NK8 (3DS1) | GGAACAGAA-2bp-GTGAACAG | 50°C/20pm | Ex 8 31-49 |
| | NK3 (2DL3) | CTGGTGCTGCAACAAA | 48°C/20pm | Ex 7 86-110 |
| | NK10 (2DL4) | GCCTGCGGGACACAGAAC | 60°C/20pm | Ex 8 23-40 |
| | NK6 (3DL1) | CAGAACAGCCAACAGCGA | 56°C/20pm | Ex 8 35-52 |
| | | | | |
| HLA-C | C293 (HLA-C 1) | ACCGAGTGAGCCTGCG | 54°C/20pm | Ex 2 220-235 |
| | C291 | (HLA-C2)TGACCGAGTGAACCTGC | 54°C/25pm | Ex 2 218-234 |

### HLA-C genotyping

HLA-C genotyping was performed using the PCR-SSOP method. Genomic DNA was amplified by PCR using the HLA-C generic primers described by Cereb N, Maye P, Lee S, Yang SY. Locus specific amplification of HLA class I genes from genomic DNA: locus specific sequences in the first and third introns of HLA-A, -B, and -C alleles. Tissue Antigens 1995;45:1-11, Table 2. Each 100µl reaction contained 0.1µg DNA, 10 µl 10 x reaction buffer, 1.5 mM MgCl₂, 0.2 mM dNTP, 0.2 µM of each primer and 2 U *Taq* DNA polymerase (Bioline, Ireland). Amplified products (937 bp) were confirmed through 1.5 % agarose gel electrophoresis. A modified version of the HLA-C typing method was used to define the HLA-C1 and C2 groups using probe C293 and C291 respectively (see Table 3).

### Statistical analysis

Gene frequencies were compared by direct counting, and analysed through SPSS Fisher exact testing. A student's independent t-test was used to compare DAS28 score means.

### Examples

The following examples are described herein so as to provide those of ordinary skill in the art with a complete disclosure and description of the invention, and are intended to be purely exemplary of the present invention, and are not intended to limit the scope of the invention.

### Example 1. Genotypes

The frequency of each KIR gene carried in the RA population was compared to a control population to look for any association of KIR genotypes with RA. The frequencies are compared in Table 4. KIR2DL2 and KIR2DS2 were carried at a higher frequency in the RA population compared to controls (51.4 % versus 40.0 %) although this was not statistically significant (NS). In general, the RA population showed little variation in frequency for each KIR compared to healthy controls, and no statistically significant differences between the two groups were seen.

**Table 4. KIR gene content frequencies compared in rheumatoid arthritis (RA) patients and healthy controls.**

| | **Frequency (%)** | |
|---|---|---|
| **KIR** **Gene** | **RA** **(n = 70)** | **Control** **(n = 100)** |
| 2DS2 | 51.4 | 40.0 |
| 2DL2 | 51.4 | 40.0 |
| 2DL3 | 94.3 | 90.0 |
| 2DL1 | 97.1 | 98.0 |
| 3DL1 | 92.9 | 94.0 |
| 3DS1 | 37.1 | 41.0 |
| 2DL5 | 51.4 | 48.0 |
| 2DS3 | 24.3 | 23.0 |
| 2DS5 | 28.6 | 36.0 |
| 2DS1 | 37.1 | 41.0 |
| 2DS4 | 98.6 | 94.0 |
| 3DL3 | 100.0 | 100.0 |
| 2DL4 | 100.0 | 100.0 |
| 3DL2 | 100.0 | 100.0 |

### Example 2. Genotypes identified in the RA population and their frequencies are compared to those of healthy controls.

A summary of the KIR genotypes and assigned haplotypes in 70 RA patients is shown in Table 5. KIR genes are arranged as they occur at the KIR locus from centromeric to telomeric positions (from left to right in Table 5) anchored at KIR2DL4. A filled box indicates the presence of a gene, an empty box represents its absence. Genotypes are arranged in order of decreasing prevalence (n) and their frequencies are compared to healthy control data.

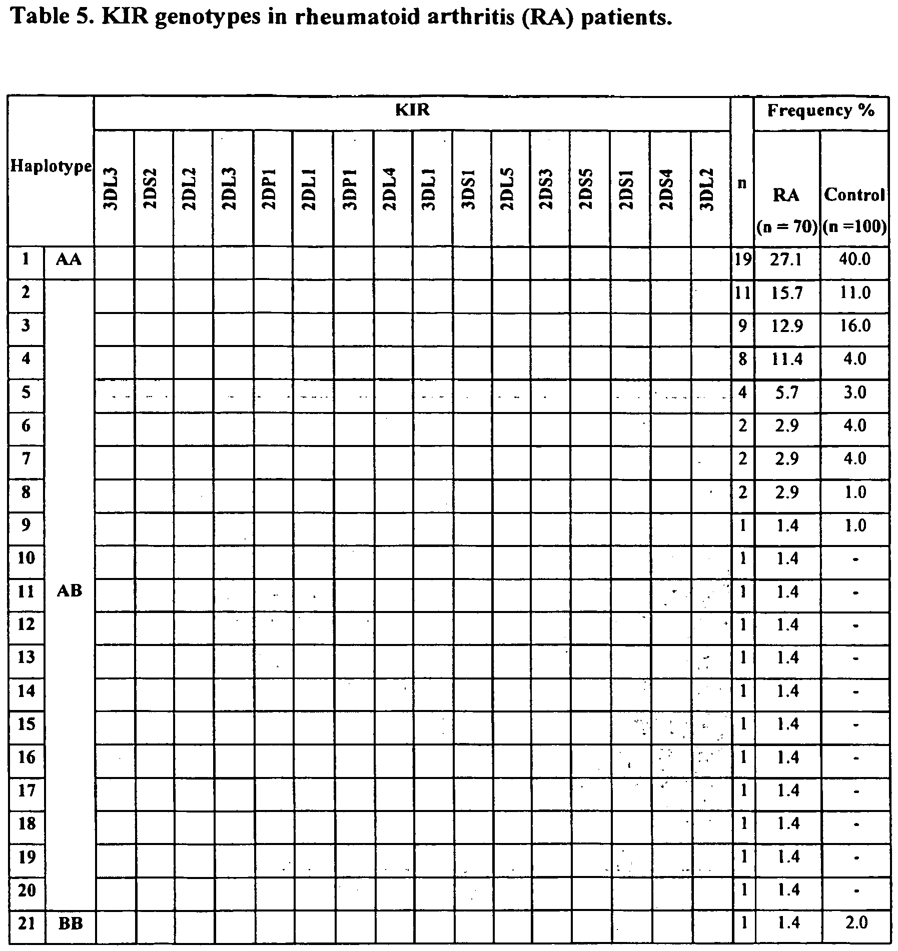

The most common genotype consisted of KIR2DL3, KIR2DP1, KIR2DL1, KIR3DL1 and KIR2DS4 with framework genes KIR2DL4, KIR3DL2, KIR3DL3, and KIR3DP1 (27.1 % RA versus 40.0 %controls; *P* = NS). This genotype corresponds to homozygosity for the AA haplotype carrying one activatory KIR, KIR2DS4. Observed in 11 of 70 patients and I 1 of 100 controls was the genotype KIR2DS2, KIR2DL2, KIR2DL3, KIR2DP1, KIR2DL1, KIR3DL1 and KIR2DS4 (15.7 % RA versus 11.0 % controls; *P* = NS). The genotype KIR2DL3, KIR2DP1, KIR2DL1, KIR3DL1, KIR3DS1, KIR2DL5, KIR2DS5, KIR2DS1, KIR2DS4 was observed in 9 of 70 patients and 16 of 100 controls (12.9 % RA versus 16.0 % controls; *P* = NS). A fourth genotype, composed of K1R2DS2, KIR2DL2, KIR2DL3, KIR2DP1, KIR2DL1, KIR3DL1, KIR2DL5, KIR2DS3 and KIR2DS4 was carried by 11.4 % of patients and 4.0 % of controls (*P* = NS). In total, 21 different genotypes were found in the RA population, compared to 19 in the controls. In the RA group, 8 of 21 genotypes were represented more than once and accounted for 81.4 % of all genotyped patients. This is significantly different to the control population where 12 of 19 genotypes were represented more than once incorporating 93.0 % of control samples, (81.4 % RA versus 93.0 % controls; *P* = 0.029). There were therefore more distinct genotypes observed in the RA population than expected.

### Example 3. Haplotypes

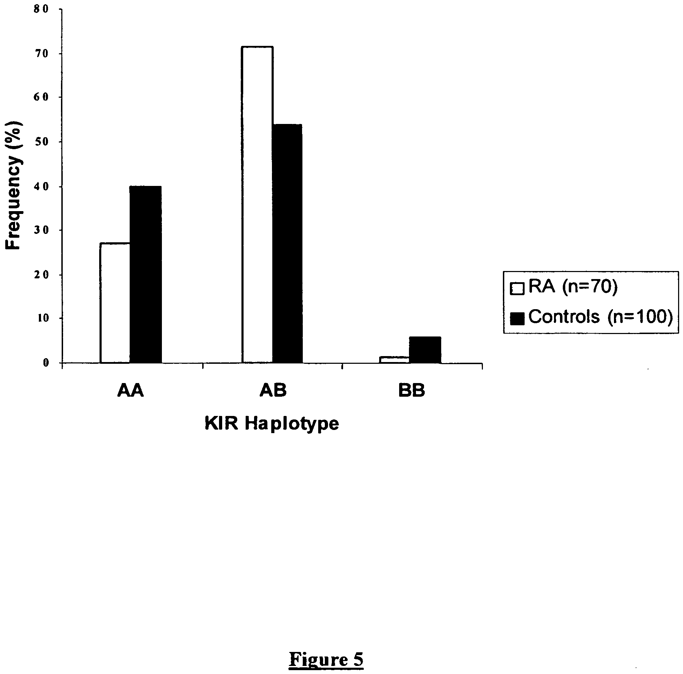

The genotypes of the RA group were arranged into the three major haplotypes AA, AB and BB according to their gene content, and compared to controls (Figure 5). In the RA population, the AA haplotype was present at a frequency of 27.1 %, AB; 71.4 % and BB; 1.4 %. The frequency of the AA and BB haplotypes in the RA patients, were not significantly different compared to controls (40.0 % and 6.0 %, respectively). The AB haplotype was significantly more frequent in the RA population (71.4 % RA versus 54.0 % controls; *P* = 0.029). The prevalence of the AB haplotype in the RA population demonstrates an increased carriership of more than one activatory KIR in the patient population.

### Example 4. HLA

To determine the functional relevance of carrying any KIR within a genotype, its putative HLA ligand must also be considered. RA patients were typed for HLA-C1 (ligand to KIR2DL2, KIR2DL3 and KIR2DS2) and HLA-C2 (ligand to KIR2DL1, KIR2DS1) groups and compared to controls. It is currently accepted that any KIR lacking its cognate HLA-ligand is essentially non-functional. Each KIR-HLA relationship (with and without the respective ligand) was compared and no significant differences were observed between the RA and control population, see Table 6. The interaction of KIR-HLA combinations in situations which may infer the most activation or inhibition were also examined and are described in Table 6. Collectively this analysis indicates a general trend towards less inhibition and more activation ofNK cells in the RA population.

**Table 6. KIR and HLA-C combinations in rheumatoid arthritis (RA) patients and healthy controls.**

| HLA-C group 1 and group 2 types are described in Table 6 as C1 and C2 respectively. In all cases, excepting those specifically labelled homozygous (homo), patients had at least one copy of the HLA-C group. | | | |
|---|---|---|---|
| | **Frequency (%)** | | |
| **KIR/HLA** | **RA** **(n = 70)** | **Control** **(n=100)** | **Assumed** **Phenotype** |
| 2DS2 with C1 | 48.5 | 38.0 | Activatory |
| 2DS2 without C1 | 2.9 | 2.0 | Non-functional |
| | | | |
| 2DL2 with C1 | 47.1 | 38.0 | Inhibitory |
| 2DL2 without C 1 | 4.3 | 2.0 | Non-functional |
| | | | |
| 2DL3 with C1 | 87.2 | 77.0 | Inhibitory |
| 2DL3 without C 1 | 7.1 | 13.0 | Non-functional |
| | | | |
| 2DS 1 with C2 | 24.3 | 20.0 | Activatory |
| 2DS 1 without C2 | 12.8 | 21.0 | Non-functional |
| | | | |
| 2DL1 with C2 | 58.5 | 52.0 | Inhibitory |
| 2DL1 without C2 | 38.6 | 46.0 | Non-functional |
| | | | |
| 2DL1 ⁺ 2DS1 ⁻ with C2 | 35.3 | 33.7 | Inhibitory |
| 2DL1 ⁺ 2DS1 ⁻ C2 homo | 5.9 | 8.3 | Inhibitory |
| 2DL3 ⁺ 2DL2/S2 ⁻ with C | 1 47.0 | 54.4 | Inhibitory |
| 2DL3 ⁺ 2DL2/S2 ⁻ C1 homo | 19.7 | 25.6 | Inhib (low) |
| 2DS2⁺ 2DL2 ⁺ | 51.4 | 40.0 | Activatory |
| 2SD2 ⁺ C1 homo | 44.4 | 57.5 | Activatory |
| 2DS1 ⁺ C2 homo | 3.9 | 12.2 | Activatory |

### Example 5. Individual KIR gene content

The KIR gene content of the responder and non-responder groups were compared to look for any association of KIR genotypes with response to anti-TNF-α therapy (Table 7). It was found that 67.6 % (23 of 34) of responders to therapy were carrying KIR2DS2/2DL2 in their genotype compared to just 33.3 % (10 of 30) of nonresponders (67.6 % responders versus 33.3 % nonresponders; *P* = 0.01). KIR2DS2/2DL2 is therefore significantly more frequently associated with responders to therapy. When compared to healthy controls, the KIR2DS2/2DL2 positive genotype remained significantly more frequent in the responder group (67.6 % responders versus 40.0 % controls; *P* = 0.009).

**Table 7. KIR gene content in responders and nonresponders to anti-TNF-a treatment. P-values are derived from the Fisher exact statistical test.**

| **Gene Responders (n = 34)** | **Nonresponders (n = 30)** | **Odds ratio** | ***P*-value** |
|---|---|---|---|
| 2DS2 23 (67.6%) | 10 (33.3 %) | 4.18 | 0.012 |
| 2DL2 23 (67.6 %) | 10 (33.3 %) | 4.18 | 0.012 |
| 3DS1 10 (29.4 %) | 15 (50.0 %) | 0.42 | 0.125 |
| 2DS5 7 (20.6 %) | 11 (36.7 %) | 0.45 | 0.175 |
| 2DS1 10 (29.4 %) | 14 (46.6 %) | 0.48 | 0.199 |
| 3DL1 33 (97.0 %) | 27 (90.0 %) | 3.67 | 0.333 |
| 2DS3 11 (32.3 %) | 6 (20.0 %) | 1.91 | 0.396 |
| 2DL5 19 (55.9 %) | 16 (53.3 %) | 1.11 | 0.413 |
| 2DS4 34 (100.0%) | 29 (96.7 %) | N/a | 0.469 |
| 2DL3 32 (94.1 %) | 29 (96.7 %) | 0.55 | 1 |
| 2DL1 33 (97.0 %) | 30 (100.0 %) | N/a | 1 |
| 3DL3 34 (100.0 %) | 30 (100.0 %) | N/a | 1 |
| 2DL4 34 (100.0%) | 30(100.0%) | N/a | 1 |
| 3DL234 (100.0 %) | 30(100.0%) | N/a | 1 |

Examination of the KIR genotypes of responders and nonresponders revealed no statistically significant differences. It is noteworthy, however, that 7 of 8 RA patients with haplotype 4 were responders (Table 5). This haplotype was also more commonly observed in the RA population when compared to controls although not statistically different. In addition, no statistically significant differences between responders and nonresponders were observed in the distribution of AA, AB and BB haplotypes. The prevalence of the AB haplotype, however, was significantly greater in the responder group (36 of 34) when compared to the control population (54 of 100) (76.5 % responders versus 54 % controls; *P* = 0.026). This finding is supportive of an activatory genotype in responders to therapy.

### Example 6. Effect of HLA-C zygosity on clinical response

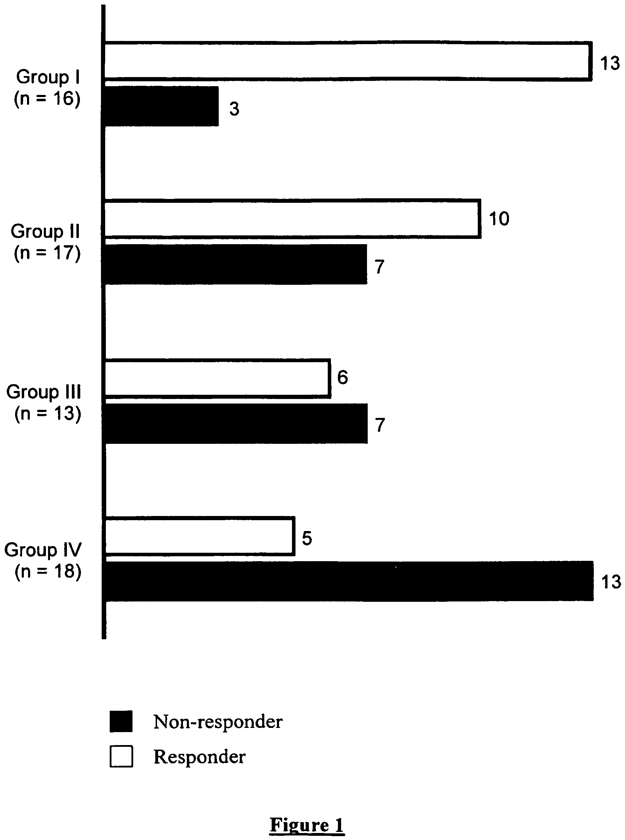

To consider the additional effect of HLA-C zygosity, patients were categorized into four groups similar to a model proposed by Nelson GW, Martin MP, Gladman D, Wade J, Trowsdale J, Carrington M. Heterozygote advantage in autoimmune disease: Hierarchy of protection/susceptibility conferred by HLA and killer Ig-like receptor combinations in Psoriatic arthritis. J Immunol 2004; 173:4273-6., ranging from most activation (group I) to most inhibition (group IV) of NK cells based on KIR-HLA interactions. Nelson's model considered the presence/absence of both KIR2DS1 and KIR2DS2 with HLA-C zygosity. However, since KIR2DS1 was not informative in this study, Nelson's model was modified to consider only KIR2DS2 in the interpretation. Thus, the most activatory genotype, group I, included patients who were KIR2DS2 positive and were HLA-C homozygous (C1/C1 or C2/C2). Patients in group II were KIR2DS2 positive and were HLA-C heterozygous (i.e., they had both ligands, C1/C2). Group III were KIR2DS2 negative and HLA-C homozygous. Finally, the most inhibitory genotype group IV, were KIR2DS2 negative and HLA-C heterozygous. When categorised in this way the ratio of responders to nonresponders inverted from group I to IV (Figure 1).

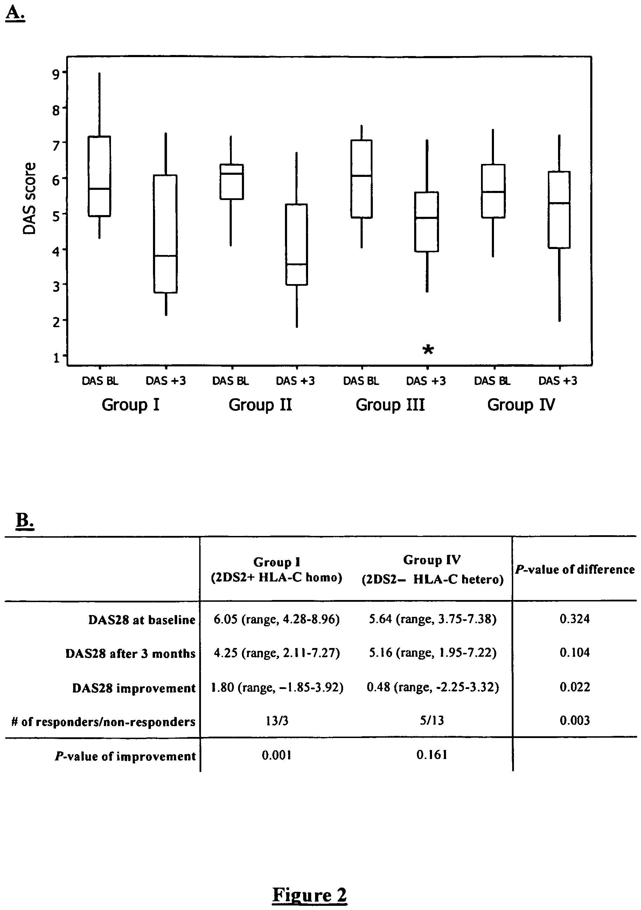

A group-wise comparison of the number of responders and non-responders revealed a significant difference between group I (13 responders, 3 non-responders) and IV (5 responders, 13 non-responders) (*P* = 0.018, adjusted for multiple comparison using Bonferroni's correction). No other group-wise comparison revealed a significant difference. The DAS28 score at baseline and after 3 months treatment was compared between the groups to further clinically support the genetic relationship of KIR2DS2 with response to therapy. There was no significant difference between the groups with respect to the DAS28 score before therapy. The majority of patients in groups I to III experienced a significant reduction of the DAS28 score after treatment (*P* = 0.001 and *P* = 0.001, and *P* = 0.011, respectively), whereas patients in group IV did not (*P* = 0.161). Figure 2B summarizes the DAS28 scores at baseline, after 3 months therapy, and the score improvements in group I and IV. The mean DAS28 score improvement for patients in group I was significantly larger than that of group IV (*P* = 0.022).

Initial examination of KIR gene content in patients revealed that carriership of both KIR2DS2 and KIR2DL2 are associated with good response to therapy. KIR2DS2 and KIR2DL2 are known to share positive linkage disequilibrium, and one is rarely found on a genotype without the other. Previous associations of KIR2DS2 in vascular complications of RA would suggest that carriership of this gene is of most relevant clinical interest. The general inversion of the numbers of responders and non-responders moving through groups I to IV also indicates a definitive role for KIR2DS2 and HLA-C zygosity in response to therapy. This study shows that an activatory KIR genotype, which could lower the activation threshold for NK and T-cell subsets, is associated with a positive clinical outcome. Previous associations of inflammatory cytokine polymorphisms such as the TNFα -308G or -857T alleles indicated that a genetic predisposition towards a proinflammatory immune response may be associated with a poor anti-TNFα therapy outcome. The functional significance of these SNPs in RA however, remains unclear and the strong linkage disequilibrium throughout the MHC region complicates the association of these and additional HLA region studies with response to therapy. It is possible that KIR2DS2 is simply in linkage with an actual 'response gene', however the documented functional associations of KIR2DS2 with RA would indicate a definitive role for this gene in response to anti-TNF-α therapy.

### Example 7. Effect of HLA-C zygosity on disease status

Figure 2A illustrates the DAS28 scores at baseline (BL) and 3 months after therapy (+3) of patients in each of groups I-IV. There was no significant difference between the groups with respect to the DAS28 score before therapy. The majority of patients in groups I to III experienced a significant reduction of the DAS28 score after treatment (*P* = 0.001, *P* = 0.001, and *P* = 0.011, respectively), whereas patients in group IV did not. Figure 2B summarises the DAS28 scores at baseline, 3 months after therapy, and the improvements in group I and IV. The mean DAS28 score improvement for patients in group I was significantly larger than that of group IV (*P* = 0.022).

These results suggest that there exist subgroups of patients characterized by distinct genotype profiles that are associated with a significant difference of response to anti-TNFα therapy. In this study, the baseline DAS28 scores of responders were significantly higher than those of non-responders, but this could not be attributed to carriership of KIR2DS2. The baseline DAS28 scores for patients with and without KIR2DS2 were not statistically different, however the improvement of the score was significantly larger in the group of patients carrying this genotype. It is possible that responders could have a different mechanism of disease pathogenesis than non-responders. The occurrence of an inhibitory profile ofNK cells in non-responders is supported by their prevalence in the proposed group IV cohort that are KIR2DS2 negative and HLA-C heterozygous.

### Example 8. The use of 2DS2 as a classifier

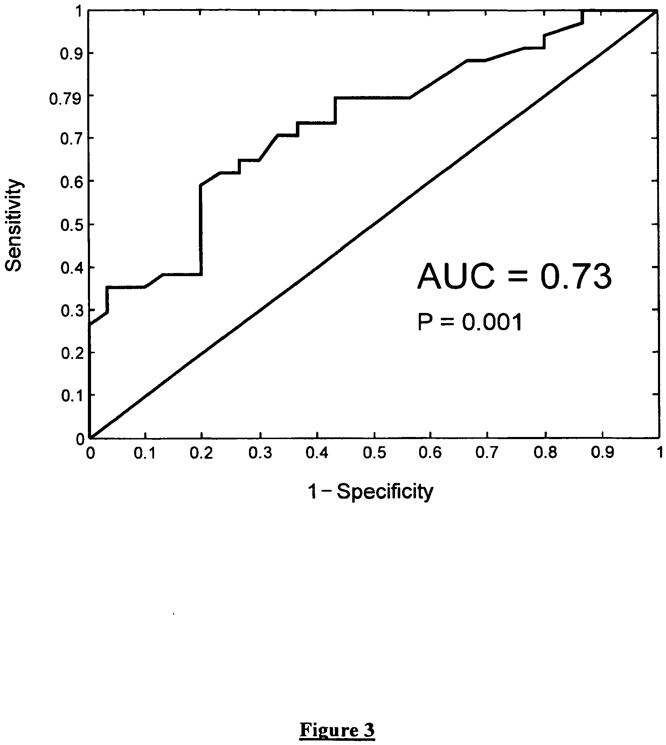

To assess whether a patient's response to treatment can be predicted, various statistical and machine learning classifiers were used, including Fisher's linear discriminant analysis (LDA), decision trees, neural networks, partial least squares and support vector machines. The performance of the models was compared using leave-one-out cross-validation (LOOCV). Of particular importance is the gene KIR2DS2, which provides the most information for predicting response to therapy. Responders showed a significantly higher frequency of KIR2DS2 (*P* = 0.012). Using only this gene and the ligand information, in addition to the patients' DAS28 scores before treatment, LDA achieved the highest overall cross-validated accuracy of 44 of 64 correct predictions (accuracy 0.69, 95%-confidence interval [0.57-0.81]). This classifier produces a posterior probability for response/non-response for each patient X, with p(responder |X) = 1 - *p*(non-responder |X). A patient X may be classified as a responder if *p*(responder|X) > *p*(non-responder|X). Referring to Figure 3, the resulting area under the ordinary receiver operating characteristic (ROC) curve is AUC = 0.73 (95%-CI [0.61-0.86]), which is a significant result (*P* = 0.001).

### Example 9. Decision support model

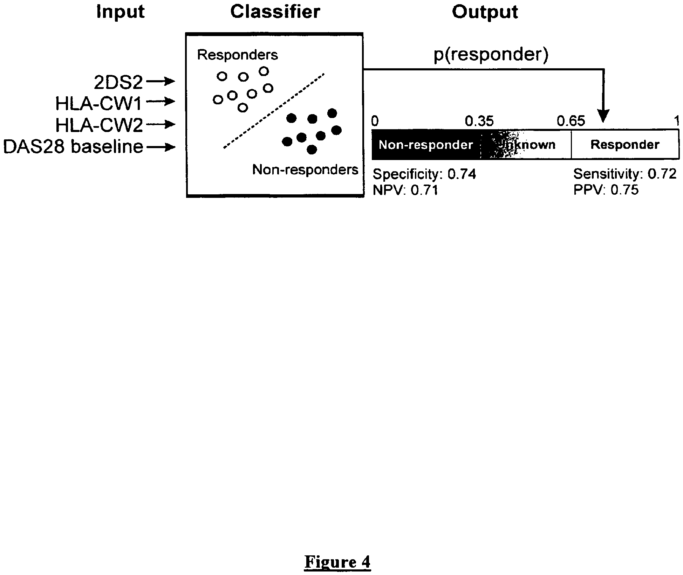

In a clinical decision process, it would be desirable to minimize the number of inconclusive cases while, at the same time, maximizing the confidence in those clinical decisions that are made. Using ROC analysis, *p* = 0.65 was identified as an optimal threshold: If a patient is predicted to respond with at least 0.65 probability, then a clinical decision is taken that s/he will respond. If a patient is predicted to respond with 0.35 probability or less, then a clinical decision is taken that s/he will not respond. In all other cases (e.g., probabilities in the range 0.35-0.65), no decision is made. This strategy is summarized in the decision support model depicted in Figure 4, which encapsulates the linear discriminant classifier.

The model takes as input information about a patient's *2DS2* status including the ligand information and the DAS28 score before treatment. For each patient, the encapsulated classifier calculates the posterior probability that s/he will respond to treatment. Only if this value exceeds the critical threshold values, then a decision is made. Using this strategy, the model predicts the outcome of 48 (75%) patients and makes no decision for 16 (25%). For those 75% of patients that the model makes in fact a prediction, an overall accuracy of 0.73, a sensitivity of 0.72, a specificity of 0.74, a positive predictive value of 0.75 and a negative predictive value of 0.71 are observed.

Particularly, it is noted that there are 11 responders of group I for which the model makes a decision, all of them being correct. There are 11 non-responding patients in group IV for which the model makes a decision; again, all being correct. Furthermore, it is noted that all predictions made with very high confidence are correct. For example, this cohort contains four responding and four non-responding patients for whom a prediction is made with more than 0.89 probability. All these high-confidence predictions are correct.

In summary, this study demonstrates a positive association of KIR2DS2 and HLA-C zygosity with good response to anti-TNFα therapy. A decision support model was built that is able to make three types of decisions: (a) a patient is a *responder* (with probability *p* ≥ 0.65) (b) a patient is a *non-responder* (with probability *q* = 1 - *p* ≥ 0.65), or (c) *unknown response* (with probability for response 0.35 < *p* < 0.65). Based on these probabilities, patients can be ranked and prioritised for treatment. The decision support model achieves high sensitivity and specificity by taking the individual genetic characteristics into account and could represent a first step towards a personalised medicine in chronic inflammatory diseases.

This predictive tool finds wide clinical utility in the management of RA, and greatly assists clinicians in the prioritisation of patients for TNF-based therapy, thereby significantly reducing the cost of treatment in terms of adverse side effects and health budgets.

## Claims

1. A method of identifying subjects likely to respond to anti-tumour necrosis factor-alpha-based therapy, the method comprising the steps of:
a. evaluating the presence, absence, or quantitative level of at least one *KIR* gene selected from *2DS2* and *2DL2*, for a subject; or evaluating the presence, absence, or quantitative level of an expression product encoded by the gene;
b. evaluating the presence, absence, or quantitative level of a gene encoding a KIR ligand selected from HLA-C Group 1, and HLA-C Group 2; or evaluating the presence, absence, or quantitative level of an expression product encoded by the gene; and
c. stratifying the subject based on the presence, absence, or quantitative level of the at least one *KIR* gene or based on the presence, absence, or quantitative level of an expression product encoded by the gene; and the presence, absence, or quantitative level of the gene encoding the KIR ligand or based on the presence, absence, or quantitative level of an expression product encoded by that gene.

2. A method according to Claim 1, wherein the presence, absence, or quantitative level of the at least one *KIR* gene is evaluated by evaluating presence, absence, or quantitative level of the expression product of the at least one *KIR* gene.

3. A method according to Claim 1 or 2, wherein the presence, absence, or quantitative level of the gene encoding a KIR ligand is evaluated by evaluating the presence, absence, or quantitative level of expression product of the gene encoding the KIR ligand.

4. A method according to Claim 2, wherein the expression product is a polypeptide encoded by the at least one *KIR* gene.

5. A method according to Claim 3, wherein the expression product is a polypeptide encoded by the gene encoding the KIR ligand.

6. A method according to any preceding claim, wherein the anti-tumour necrosis factor-alpha-based therapy is a therapeutic intervention targeting tumour necrosis factor-alpha signalling.

7. A method according to any preceding claim, wherein the at least one *KIR* gene is *2DS2*, and wherein the KIR ligand is HLA-C Group 1.

8. A method according to Claim 1, wherein the method further comprises the step of determining the haplotype of the subject.

9. A method according to Claim 8, wherein the presence of a Group B haplotype is indicative that a subject will respond (is a responder).

10. A method according to any preceding claim, wherein the presence of at least one copy of *2DS2*, and HLA-C Group I homozygosity (CI/CI) is indicative that a subject will respond (is a responder).

11. A method according to any preceding claim, wherein the presence of at least one copy of *2DS2*, and HLA-C Group 2 homozygosity (C2/C2) is indicative that a subject will respond (is a responder).

12. A method according to any preceding claim, wherein the method further comprises the step of evaluating the DAS28 score of the subject.

13. Use of at least one *Killer cell immunoglobulin-like receptor* gene selected from *2DS2* and *2DL2* and a gene encoding a KIR ligand selected from HLA-C Group 1, and HLA-C Group 2for the identification of subjects likely to respond to anti-tumour necrosis factor-alpha-based therapy.

## Patentansprüche

1. Verfahren zur Identifizierung von Subjekten, die wahrscheinlich auf Anti-Tumor-Nekrose-Faktor-alpha-basierte Therapie ansprechen, wobei das Verfahren die folgenden Schritte umfasst:
a. Bewertung der Gegenwart, Abwesenheit oder quantitativen Menge von mindestens einem *KIR*-Gen, das aus *2DS2* und *2DL2* ausgewählt ist, für ein Subjekt; oder Bewertung der Gegenwart, Abwesenheit oder quantitativen Menge eines Expressionsprodukts, das durch das Gen kodiert wird;
b. Bewertung der Gegenwart, Abwesenheit oder quantitativen Menge eines Gens, das einen KIR-Liganden kodiert, der aus HLA-C Gruppe 1 und HLA-C Gruppe 2 ausgewählt ist; oder Bewertung der Gegenwart, Abwesenheit oder quantitativen Menge eines Expressionsprodukts, das durch das Gen kodiert wird; und
c. Stratifizierung des Subjekts auf der Grundlage der Gegenwart, Abwesenheit oder quantitativen Menge des mindestens einen *KIR*-Gens oder auf der Grundlage der Gegenwart, Abwesenheit oder quantitativen Menge eines Expressionsprodukts, das durch das Gen kodiert wird; und der Gegenwart, Abwesenheit oder quantitativen Menge des Gens, das den KIR-Liganden kodiert, oder auf der Grundlage der Gegenwart, Abwesenheit oder quantitativen Menge eines Expressionsprodukts, das durch dieses Gen kodiert wird.

2. Verfahren nach Anspruch 1, worin die Gegenwart, Abwesenheit oder quantitative Menge des mindestens einen *KIR*-Gens durch die Bewertung der Gegenwart, Abwesenheit oder quantitativen Menge des Expressionsprodukts des mindestens einen *KIR*-Gens bewertet wird.

3. Verfahren nach Anspruch 1 oder 2, worin die Gegenwart, Abwesenheit oder quantitative Menge des Gens, das einen KIR-Liganden kodiert, durch die Bewertung der Gegenwart, Abwesenheit oder quantitativen Menge des Expressionsprodukts des Gens, das den KIR-Liganden kodiert, bewertet wird.

4. Verfahren nach Anspruch 2, worin das Expressionsprodukt ein Polypeptid ist, das durch das mindestens eine *KIR*-Gen kodiert wird.

5. Verfahren nach Anspruch 3, worin das Expressionsprodukt ein Polypeptid ist, das durch das Gen kodiert wird, das den KIR-Liganden kodiert.

6. Verfahren nach einem vorstehenden Anspruch, worin die Anti-Tumor-Nekrose-Faktor-alpha-basierte Therapie eine therapeutische Intervention ist, die auf die Tumor-Nekrose-Faktor-alpha-Signalgebung abzielt.

7. Verfahren nach einem vorstehenden Anspruch, worin das mindestens eine *KIR-*Gen *2DS2* ist und worin der KIR-Ligand HLA-C Gruppe 1 ist.

8. Verfahren nach Anspruch 1, worin das Verfahren weiter den Schritt der Bestimmung des Haplotyps des Subjekts umfasst.

9. Verfahren nach Anspruch 8, worin die Gegenwart eines Gruppe-B-Haplotyps anzeigt, dass ein Subjekt ansprechen wird (ein Responder ist).

10. Verfahren nach einem vorstehenden Anspruch, worin die Gegenwart von mindestens einer Kopie von *2DS2* und HLA-C-Gruppe-1-Homozygotie (C1/C1) anzeigt, dass ein Subjekt ansprechen wird (ein Responder ist).

11. Verfahren nach einem vorstehenden Anspruch, worin die Gegenwart von mindestens einer Kopie von *2DS2* und HLA-C-Gruppe-2-Homozygotie (C2/C2) anzeigt, dass ein Subjekt ansprechen wird (ein Responder ist).

12. Verfahren nach einem vorstehenden Anspruch, worin das Verfahren weiter den Schritt der Bewertung des DAS28-Werts des Subjekts umfasst.

13. Verwendung von mindestens einem *Killerzell-Immunoglobulin-ähnlichen-Rezeptor*-Gen, das aus *2DS2* und *2DL2* ausgewählt ist, und einem Gen, das einen KIR-Liganden kodiert, der aus HLA-C Gruppe 1 und HLA-C Gruppe 2 ausgewählt ist, zur Identifizierung von Subjekten, die wahrscheinlich auf Anti-Tumor-Nekrose-Faktoralpha-basierte Therapie ansprechen.

## Revendications

1. Procédé d'identification de sujets susceptibles de répondre à une thérapie basée sur un anti-facteur de nécrose tumorale-alpha, le procédé comprenant les étapes consistant à :
a. évaluer, pour un sujet, la présence, l'absence ou le niveau quantitatif d'au moins un gène *KIR* choisi parmi *2DS2* et *2DL2* ; ou évaluer la présence, l'absence ou le niveau quantitatif d'un produit d'expression codé par le gène ;
b. évaluer la présence, l'absence ou le niveau quantitatif d'un gène codant pour un ligand KIR choisi parmi HLA-C Groupe 1 et HLA-C Groupe 2 ; ou évaluer la présence, l'absence ou le niveau quantitatif d'un produit d'expression codé par le gène ; et
c. stratifier le sujet sur la base de la présence, l'absence ou le niveau quantitatif de l'au moins un gène *KIR* ou sur la base de la présence, l'absence ou le niveau quantitatif d'un produit d'expression codé par le gène ; et la présence, l'absence ou le niveau quantitatif d'un gène codant pour le ligand KIR ou sur la base de la présence, l'absence ou le niveau quantitatif d'un produit d'expression codé par ce gène.

2. Procédé selon la revendication 1, dans lequel la présence, l'absence ou le niveau quantitatif de l'au moins un gène *KIR* est évalué en évaluant la présence, l'absence ou le niveau quantitatif du produit d'expression de l'au moins un gène *KIR.*

3. Procédé selon la revendication 1 ou 2, dans lequel la présence, l'absence ou le niveau quantitatif du gène codant pour un ligand KIR est évalué en évaluant la présence, l'absence ou le niveau quantitatif du produit d'expression du gène codant pour le ligand KIR.

4. Procédé selon la revendication 2, dans lequel le produit d'expression est un polypeptide codé par l'au moins un gène *KIR.*

5. Procédé selon la revendication 3, dans lequel le produit d'expression est un polypeptide codé par le gène codant pour le ligand KIR.

6. Procédé selon l'une quelconque des revendications précédentes, dans lequel la thérapie basée sur un anti-facteur de nécrose tumorale-alpha est une intervention thérapeutique ciblant la signalisation du facteur de nécrose tumorale-alpha.

7. Procédé selon l'une quelconque des revendications précédentes, dans lequel l'au moins un gène *KIR* est *2DS2,* et dans lequel le ligand KIR est HLA-C Groupe 1.

8. Procédé selon la revendication 1, dans lequel le procédé comprend en outre l'étape consistant à déterminer l'haplotype du sujet.

9. Procédé selon la revendication 8, dans lequel la présence d'un haplotype du Groupe B indique qu'un sujet produira une réponse (est un répondant).

10. Procédé selon l'une quelconque des revendications précédentes, dans lequel la présence d'au moins une copie de *2DS2* et du caractère homozygote à HLA-C Groupe 1 (C1/C1) indique qu'un sujet produira une réponse (est un répondant).

11. Procédé selon l'une quelconque des revendications précédentes, dans lequel la présence d'au moins une copie de *2DS2* et du caractère homozygote à HLA-C Groupe 2 (C2/C2) indique qu'un sujet produira une réponse (est un répondant).

12. Procédé selon l'une quelconque des revendications précédentes, dans lequel le procédé comprend en outre l'étape consistant à évaluer le score DAS28 du sujet.

13. Utilisation d'au moins un gène *KIR* choisi parmi *2DS2* et *2DL2* et d'un gène codant pour un ligand KIR choisi parmi HLA-C Groupe 1 et HLA-C Groupe 2 en vue de l'identification de sujets susceptibles de répondre à une thérapie basée sur un anti-facteur de nécrose tumorale-alpha.
